# EUROPÄISCHE PATENTANMELDUNG

(11) **EP 3 459 456 A1**
(43) Veröffentlichungstag der Anmeldung: **27.03.2019**
(21) Anmeldenummer: 17193253.6
(22) Anmeldetag: 26.09.2017
(51) Int. Cl.: A61B 5/113, A61B 5/00, A61B 6/00

(54) **PATIENTENSCHNITTSTELLE ZUR UNTERSTÜTZUNG EINER ATMUNG EINES PATIENTEN WÄHREND EINER MEDIZINISCHEN BILDGEBUNGSUNTERSUCHUNG**

(71) Anmelder: Siemens Healthcare GmbH, 91052 Erlangen (DE)
(72) Erfinder: Kartäusch, Ralf, 91058 Erlangen (DE); Paul, Dominik, 91088 Bubenreuth (DE)

(57) **Zusammenfassung**

Die Erfindung geht aus von einem Verfahren zu einer Unterstützung einer Atmung eines Patienten während einer medizinischen Bildgebungsuntersuchung mittels einer medizinischen Bildgebungsvorrichtung umfassend die folgenden Schritte:
- Erfassen eines Atemsignals des Patienten während der medizinischen Bildgebungsuntersuchung,
- Ermitteln einer Abweichung des erfassten Atemsignals von einem idealen Atemzustand für die medizinische Bildgebungsvorrichtung,
- Generieren einer Feedbackinformation für den Patienten anhand der Abweichung und
- Ausgabe der Feedbackinformation an den Patienten mittels einer Patientenschnittstelle während der medizinischen Bildgebungsuntersuchung.

## Beschreibung

Die vorliegende Erfindung betrifft ein Verfahren zu einer Unterstützung einer Atmung eines Patienten während einer medizinischen Bildgebungsuntersuchung mittels einer medizinischen Bildgebungsvorrichtung. Des Weiteren betrifft die vorliegende Erfindung eine medizinische Bildgebungsvorrichtung mit einer Patientenschnittstelle, wobei die medizinische Bildgebungsvorrichtung zusammen mit einer Steuereinheit zu einer Ausführung des Verfahrens zu einer Unterstützung einer Atmung eines Patienten während einer medizinischen Bildgebungsuntersuchung ausgelegt ist.

Bei medizinischen Bildgebungsuntersuchungen, wie beispielsweise einer Magnetresonanzuntersuchung, kann es erforderlich sein, die Atmung des Patienten auf die medizinische Bildgebungsuntersuchung abzustimmen. Dies ist beispielsweise für Untersuchungen des Abdomens oder des Herzens des Patienten der Fall. Hierbei ist es oft erforderlich, dass die erfassten Bilddaten stets in einer gleichen Atemphase, wie beispielsweise stets im ausgeatmeten Zustand des Patienten oder im eingeatmeten Zustand des Patienten usw. erfasst werden. Insbesondere langes Atemanhalten oder auch ein Atemstopp kann dabei zu Fehlern in der Messung führen.

Bisherige Methoden, um bei freier Atmung des Patienten medizinische Bildgebungsuntersuchungen von beispielsweise des Herzens oder des Abdomens des Patienten durchführen zu können, greifen auf Triggersignale zurück, die aufgrund eines Atemsignals des Patienten generiert wurden und die eine Messung auslösen. Diese Triggersignale lösen jedoch nur bei einem bestimmten Atemzustand des Patienten aus. Auch können Bewegungen, wie insbesondere Atembewegungen, in den erfassten Messdaten im Nachhinein korrigiert werden.

Jedoch funktionieren diese Methoden nur zufriedenstellend, wenn der Patient möglichst periodische Atembewegungen ausführt. Sobald der Patient nicht periodische Atembewegungen ausführt, kann dies zu Problemen bei der Triggererkennung führen. Dies hat insbesondere eine reduzierte Bildqualität zur Folge. Zusätzlich kann dies auch zu einem Abbruch der Messung und/oder der medizinischen Bildgebungsuntersuchung führen, die dann wiederholt werden muss, was besonders zeitaufwendig ist. Zudem ist eine sofortige Wiederholung der medizinischen Bildgebungsuntersuchung bei einer vorherigen Kontrastmittelgabe nicht möglich.

Aus der DE 10 2014 202 606 A1 ist beispielsweise ein Verfahren zur Aufnahme von Magnetresonanzdaten bekannt.

Der vorliegenden Erfindung liegt insbesondere die Aufgabe zugrunde, den Patienten derart zu unterstützen, dass der Patient seine Atmung an die medizinische Bildgebungsuntersuchung anpassen kann. Die Aufgabe wird durch die Merkmale der unabhängigen Ansprüche gelöst. Vorteilhafte Ausgestaltungen sind in den Unteransprüchen beschrieben.

Die Erfindung geht aus von einem Verfahren zu einer Unterstützung einer Atmung eines Patienten während einer medizinischen Bildgebungsuntersuchung mittels einer medizinischen Bildgebungsvorrichtung umfassend die folgenden Schritte:
- Erfassen eines Atemsignals des Patienten während der medizinischen Bildgebungsuntersuchung,
- Ermitteln einer Abweichung des erfassten Atemsignals von einem idealen Atemzustand für die medizinische Bildgebungsuntersuchung,
- Generieren einer Feedbackinformation für den Patienten anhand der Abweichung und
- Ausgabe der Feedbackinformation an den Patienten mittels einer Patientenschnittstelle während der medizinischen Bildgebungsuntersuchung.

Die medizinische Bildgebungsuntersuchung kann von allen dem Fachmann als sinnvoll erscheinenden medizinischen Bildgebungsuntersuchungen gebildet sein, wie beispielsweise eine Computertomografie-Untersuchung und/oder eine Röntgenuntersuchung. Vorzugsweise jedoch umfasst die medizinische Bildgebungsuntersuchung eine Magnetresonanzuntersuchung, da hier aufgrund der langen Untersuchungszeiten eine korrekte Atmung des Patienten besonders vorteilhaft ist. Die medizinische Bildgebungsvorrichtung kann demnach auch von allen dem Fachmann als sinnvoll erscheinenden medizinischen Bildgebungsvorrichtungen gebildet sein, wie beispielsweise von einer Computertomografie-Vorrichtung und/oder von einer Röntgenvorrichtung usw. gebildet sein. Besonders vorteilhaft jedoch umfasst die medizinische Bildgebungsvorrichtung eine Magnetresonanzvorrichtung.

Die medizinische Bildgebungsuntersuchung kann zudem mehrere Messungen umfassen zur Erfassung und/oder Messung von medizinischen Bilddaten, insbesondere Rohbilddaten. Eine von einer Magnetresonanzuntersuchung gebildete medizinische Bildgebungsuntersuchung kann vorzugsweise mehrere unterschiedliche Sequenzen und/oder Protokolle umfassen, beispielsweise zur Erzeugung von unterschiedlichen Kontrasten und/oder zur Erzeugung von unterschiedlichen Ansichten in den erfassten Rohbilddaten.

Die Patientenschnittstelle ist zu einem Austausch und/oder eine Kommunikation zwischen der medizinischen Bildgebungsvorrichtung, insbesondere einer Steuereinheit der medizinischen Bildgebungsvorrichtung, und dem Patienten ausgelegt. Zudem kann es auch sein, dass mittels der Patientenschnittstelle der Patient auch mit einem medizinischen Bedienpersonal kommunizieren kann. Die Patientenschnittstelle kann eine akustische und/oder auditive Patientenschnittstelle umfassen. Alternativ oder zusätzlich kann die Patientenschnittstelle auch eine bildliche und/oder visuelle Patientenschnittstelle umfassen. Die akustische und/oder auditive Patientenschnittstelle kann beispielsweise Kopfhörer umfassen, die der Patient während der medizinischen Bildgebungsuntersuchung trägt. Zudem kann die akustische Patientenschnittstelle auch weitere akustische Ausgabeeinheiten umfassen, wie beispielsweise Lautsprecher. Die bildliche und/oder visuelle Patientenschnittstelle kann beispielsweise ein Display und/oder einen Monitor umfassen, das vorzugsweise in einem voraussichtlichen Sichtfeld des Patienten während der medizinischen Bildgebungsuntersuchung angeordnet ist. Vorzugsweise jedoch ist das Display und/oder der Monitor außerhalb des Patientenaufnahmebereichs angeordnet und wird mittels eines Spiegels in den Patientenaufnahmebereich hineinprojiziert. Zudem kann das Display und/oder der Monitor auch direkt an einer dem Patienten gegenüberliegenden Wand eines Patientenaufnahmebereichs der medizinischen Bildgebungsvorrichtung angeordnet sein. Zudem kann die bildliche und/oder visuelle Patientenschnittstelle auch eine Videobrille und/oder eine Spiegelbrille und/oder eine Projektionseinheit mit einer Projektionsfläche innerhalb des Patientenaufnahmebereichs der medizinischen Bildgebungsvorrichtung umfassen.

Die Feedbackinformation an den Patienten kann eine akustische und/oder auditive Feedbackinformation und/oder auch eine visuelle Feedbackinformation an den Patienten umfassen.

Unter einem Atemsignal soll hierbei insbesondere ein Signal verstanden werden, anhand dessen ein Atemvorgang und/oder ein Atemzustand des Patienten charakterisiert und/oder bestimmt werden kann. Das Atemsignal kann vorzugsweise mittels eines Atemsensors erfasst werden. Zudem ist auch eine Erfassung mittels einer Navigatormessung zur Erfassung von Patientenbewegungen möglich. Das Atemsignal kann beispielsweise einen ausgeatmeten Zustand und/oder einen eingeatmeten Zustand und/oder einen Zustand eines Einatmens und/oder eines Ausatmens und/oder eine Atemfrequenz und/oder ein Atemzyklus des Patienten usw. umfassen.

Der ideale Atemzustand ist bevorzugt an die medizinische Bildgebungsuntersuchung angepasst, so dass ein optimaler Untersuchungsablauf erfolgen kann. Vorzugsweise ist der ideale Atemzustand im System, insbesondere in einer Steuereinheit und/oder der medizinischen Bildgebungsvorrichtung, hinterlegt. Der ideale Atemzustand legt bevorzugt fest, wann der Patient sich in welcher Atemphase für die medizinische Bildgebungsuntersuchung befinden sollte. Mittels der Abweichung wird ermittelt, ob die Atmung des Patienten, insbesondere das Atemsignal des Patienten, von dem idealen Atemzustand für die medizinische Bildgebungsuntersuchung abweicht. Beispielsweise wird mittels der Abweichung ermittelt, ob der Patient bezüglich des idealen Atemzustands zu schnell oder zu langsam atmet. Das Atemsignal des Patienten wird dabei kontinuierlich während der gesamten medizinischen Bildgebungsuntersuchung erfasst, so dass für den Patienten während der gesamten medizinischen Bildgebungsuntersuchung ein aktuelles Feedbacksignal zur Verfügung steht.

Mittels der Feedbackinformation kann dem Patienten übermittelt werden, wie gut seine aktuelle Atmung und/oder sein aktueller Atemzyklus zur medizinischen Bildgebungsuntersuchung passt bzw. mit dieser übereinstimmt. Ein Vorteil hiervon ist, dass der Patient während der medizinischen Bildgebungsuntersuchung, also während der laufenden Untersuchung, seine Atmung und/oder seinen Atemzustand aufgrund der Feedbackinformation an die medizinische Bildgebungsuntersuchung anpassen kann. Hierdurch kann auch eine hohe Bildqualität in den erfassten medizinischen Bilddaten erreicht werden. Zudem können aufgrund der Feedbackinformation und der Anpassung der Atmung des Patienten an die medizinische Bildgebungsuntersuchung auch eine Messzeit verkürzt werden und/oder unerwünschte Messabbrüche vermieden werden. Dabei kann auch eine Stresssituation für den Patienten reduziert werden. Zudem kann dies auch zu einer erhöhten Effizienz der medizinischen Bildgebungsvorrichtung führen.

In einer vorteilhaften Weiterbildung der Erfindung kann es vorgesehen sein, dass das Atemsignal mittels eines Atemsensors erfasst wird. Der Atemsensor kann beispielsweise einen Atemgürtel und/oder ein Atemkissen und/oder weitere, dem Fachmann als sinnvoll erscheinende Atemsensoren umfassen. Hierdurch kann eine einfache und insbesondere direkte Erfassung des Atemsignals des Patienten erreicht werden.

Alternativ oder zusätzlich kann das Atemsignal auch anhand eines empfangenen Bilddatensignals einer Navigatormessung zur Erfassung von Patientenbewegungen erfasst werden. Dies ermöglicht eine Erfassung des Atemsignals des Patienten ohne dass hierbei zusätzlich Einheiten am Patienten angebracht werden müssen, wie beispielsweise ein Atemsensor.

In einer weiteren Ausgestaltung der Erfindung kann es vorgesehen sein, dass die Feedbackinformation visuell ausgegeben wird. Derart kann eine besonders einfach erfassbare Feedbackinformation für den Patienten während der medizinischen Bildgebungsuntersuchung bereitgestellt werden. Zudem können auch hörbehinderte Patienten besonders einfach einen aktuellen Zustand ihrer Atmung in Bezug auf den idealen Atemzustand erfassen.

Alternativ oder zusätzlich kann die Feedbackinformation auch akustisch und/oder auditiv ausgegeben werden. Dies ermöglicht ebenfalls eine einfach erfassbare Feedbackinformation für den Patienten während der medizinischen Bildgebungsuntersuchung.

In einer weiteren Ausgestaltung der Erfindung kann es vorgesehen sein, dass die Feedbackinformation ein Objekt umfasst, das mittels des Atemsignals steuerbar ist. Umfasst die Feedbackinformation eine akustische und/oder auditive Feedbackinformation, so umfasst bevorzugt das Objekt ein akustisches und/oder auditives Objekt. Umfasst dagegen die Feedbackinformation eine bildliche und/oder visuelle Feedbackinformation, so umfasst bevorzugt das Objekt auch ein bildliches und/oder visuelles Objekt. In diesem Zusammenhang soll unter steuerbar insbesondere verstanden werden, dass der Patient durch Ändern seiner Atmung, insbesondere durch Ändern seines Atemzustands und/oder seines Atemzyklusses, das Objekt der Feedbackinformation steuern kann. Insbesondere kann der Patient mittels des Atemsignals eine Eigenschaft des Objekts beeinflussen. Vorzugsweise ist das Objekt derart beschaffen, dass der Patient bestrebt ist, das Objekt in einen bestimmten Zustand zu bringen. In diesem bestimmten Zustand des Objekts liegt im Wesentlichen eine Übereinstimmung des Atemsignals mit dem idealen Atemzustand vor. Dieser bestimmte Zustand umfasst somit eine minimale Abweichung zwischen dem erfassten Atemsignal und dem idealen Atemzustand. Durch diese Ausgestaltung der Erfindung kann für den Patienten eine besonders intuitive Unterstützung der Atmung während der medizinischen Bildgebungsuntersuchung bereitgestellt werden. Zudem kann auf Atemkommandos, die dem Patienten ständig einen Atemzustand mitteilen, verzichtet werden und derart zu einer entspannten Untersuchungsatmosphäre für den Patienten beigetragen werden.

In einer weiteren Ausgestaltung der Erfindung kann es vorgesehen sein, dass das Objekt der Feedbackinformation aufgrund einer Atemgeschwindigkeit des Patienten und/oder einer Atemfrequenz des Patienten und/oder eines Atemzustands des Patienten steuerbar ist. Besonders vorteilhaft kann der Patient durch bewusstes Ändern seiner Atemgeschwindigkeit und/oder seiner Atemfrequenz und/oder seines Atemzustands das Objekt der Feedbackinformation steuern und beispielsweise den bestimmten Zustand des Objekts einstellen. Insbesondere kann hierbei eine Eigenschaft des Objekts direkt vom Patienten durch Änderung seiner Atemgeschwindigkeit und/oder seiner Atemfrequenz und/oder seines Atemzustands verändert und/oder gesteuert werden.

In einer weiteren Ausgestaltung der Erfindung kann es vorgesehen sein, dass das Objekt ein bildliches und/oder visuelles Objekt umfasst, das mittels der Patientenschnittstelle darstellbar ist und dessen Aussehen und/oder dessen Geschwindigkeit und/oder dessen Größe aufgrund der Atemgeschwindigkeit des Patienten und/oder der Atemfrequenz des Patienten und/oder des Atemzustands des Patienten steuerbar ist. Dies ermöglicht eine einfache und direkte Steuerung des Objekts. Beispielsweise kann der Patient hierbei spielerisch animiert werden, einen bestimmten Zustand des Objekts zu erreichen und damit auch einen nahezu idealen Atemzustand für die medizinische Bildgebungsuntersuchung. Beispielsweise kann das Objekt ein Tier sein, dessen Geschwindigkeit und/oder dessen Position durch die Atmung des Patienten eingestellt und/oder gesteuert werden. Z.B. kann das Objekt einen Haifisch umfassen, dessen Geschwindigkeit und/oder Schwimmhöhe durch die Atmung des Patienten eingestellt und/oder gesteuert werden kann. Zudem können auch hörbehinderte Patienten besonders einfach einen aktuellen Zustand seiner Atmung in Bezug auf den idealen Atemzustand erfassen.

In einer weiteren Ausgestaltung der Erfindung kann es vorgesehen sein, dass das Objekt einen Ton und/oder eine Tonfolge umfasst, wobei eine Frequenz und/oder eine Amplitude des Tons und/oder der Tonfolge aufgrund der Atemgeschwindigkeit des Patienten und/oder der Atemfrequenz des Patienten und/oder des Atemzustands des Patienten steuerbar ist. Dies ermöglicht eine einfache und direkte Steuerung des Objekts. Beispielsweise kann der ausgegebene Ton und/oder die ausgegebene Tonfolge einen hohen Ton und/oder eine hohe Tonfolge umfassen, wenn die Atmung, insbesondere der Atemzustand des Patienten zu langsam bezüglich des idealen Atemzustands ist. Auch kann beispielsweise der ausgegebene Ton und/oder die ausgegebene Tonfolge einen tiefen Ton und/oder eine tiefe Tonfolge umfassen, wenn die Atmung, insbesondere der Atemzustand des Patienten zu schnell bezüglich des idealen Atemzustands ist. Umfasst das akustische Objekt eine Tonfolge und/oder eine Melodie, kann auch ein Takt mit dem aktuellen Atemzustand des Patienten geändert werden und/oder die eine Geschwindigkeit der Tonfolge und/oder Melodie geändert werden.

Darüber hinaus kann es vorgesehen sein, dass in Abhängigkeit des erfassten Atemsignals medizinische Bilddaten während der medizinischen Bildgebungsuntersuchung erfasst werden. In diesem Zusammenhang sollen unter erfassten medizinischen Bilddaten insbesondere Rohbilddaten verstanden werden, die mittels einer medizinischen Bildgebungsvorrichtung während der medizinischen Bildgebungsuntersuchung akquiriert werden. Vorzugsweise kann das erfasste Atemsignal als Triggersignal dienen und/oder anhand des erfassten Atemsignals ein Triggersignal generiert werden, so dass in Abhängigkeit des Triggersignals und somit in Abhängigkeit zu dem erfassten Atemsignal eine Messung ausgelöst wird.

Des Weiteren geht die Erfindung aus von einer Steuereinheit mit einer Prozessoreinheit, die zu einer Steuerung des Verfahrens zu einer Unterstützung einer Atmung eines Patienten während einer medizinischen Bildgebungsuntersuchung ausgelegt ist. Vorzugsweise weist hierzu die Steuereinheit eine Software und/oder Computerprogramme auf, die bei einem Ausführen mittels der Prozessoreinheit das erfindungsgemäße Verfahren ausführen. Die Software und/oder Computerprogramme können dabei innerhalb der Steuereinheit gespeichert sein, wobei hierzu die Steuereinheit eine Speichereinheit aufweist. Zudem können die Software und/oder Computerprogramme auch innerhalb einer Speichereinheit der medizinischen Bildgebungsvorrichtung und/oder einer externen Speichereinheit, wie beispielsweise einer Cloud, gespeichert sein. Die Steuereinheit kann zudem von der medizinischen Bildgebungsvorrichtung umfasst sein. Dabei kann beispielsweise die Steuereinheit innerhalb einer Systemsteuereinheit der medizinischen Bildgebungsvorrichtung integriert sein. Zudem kann die Steuereinheit auch eine externe Steuereinheit umfassen, die beispielsweise innerhalb einer zentralen Steuerungsvorrichtung zur Steuerung von mehreren medizinischen Bildgebungsvorrichtungen angeordnet ist und über ein Datennetz auf die medizinischen Bildgebungsvorrichtungen zugreift. Zudem kann die Steuereinheit auch von einer Cloud umfasst sein.

Vorzugsweise ist die Steuereinheit dazu ausgebildet, dass anhand des erfassten Atemsignals des Patienten die Abweichung des erfassten Atemsignals bezüglich des idealen Atemzustands ermittelt wird. Des Weiteren ist vorteilhafterweise die Steuereinheit dazu ausgebildet, dass anhand der Abweichung die Feedbackinformation generiert wird und die Feedbackinformation zur Patientenschnittstelle der medizinischen Bildgebungsvorrichtung übertragen und/oder geleitet wird.

Die Vorteile der erfindungsgemäßen Steuereinheit entsprechen im Wesentlichen den Vorteilen des erfindungsgemäßen Verfahrens zu einer Unterstützung einer Atmung eines Patienten während einer medizinischen Bildgebungsuntersuchung, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Des Weiteren geht die Erfindung aus von einer medizinischen Bildgebungsvorrichtung mit einer Scannereinheit zur Erfassung von medizinischen Bildgebungsdaten während einer medizinischen Bildgebungsuntersuchung. Zudem weist die medizinische Bildgebungsvorrichtung einen Atemsensor und eine Patientenschnittstelle auf. Die medizinische Bildgebungsvorrichtung ist zusammen mit der Steuereinheit zu einer Ausführung eines Verfahrens zu einer Unterstützung einer Atmung eines Patienten während einer medizinischen Bildgebungsuntersuchung ausgelegt.

Die Vorteile der erfindungsgemäßen medizinischen Bildgebungsvorrichtung entsprechen im Wesentlichen den Vorteilen des erfindungsgemäßen Verfahrens zu einer Unterstützung einer Atmung eines Patienten während einer medizinischen Bildgebungsuntersuchung, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Darüber hinaus kann es in einer vorteilhaften Weiterbildung der Erfindung vorgesehen sein, dass die Patientenschnittstelle eine akustische Patientenschnittstelle umfasst. Derart kann eine besonders einfach erfassbare Feedbackinformation für den Patienten während der medizinischen Bildgebungsuntersuchung bereitgestellt werden. Alternativ oder zusätzlich kann die die Patientenschnittstelle auch eine visuelle Patientenschnittstelle umfassen.

Des Weiteren geht die Erfindung aus von einem Computerprogrammprodukt, welches ein Programm umfasst und direkt in einem Speicher einer programmierbaren Steuereinheit ladbar ist, mit Programmmitteln, um ein Verfahren zu einer Unterstützung einer Atmung eines Patienten während einer medizinischen Bildgebungsuntersuchung auszuführen, wenn das Programm in der Steuereinheit ausgeführt wird. Dabei benötigt das Computerprogramm eventuell Programmmittel, z.B. Bibliotheken und Hilfsfunktionen, um die entsprechenden Ausführungsformen des Verfahrens zu realisieren. Das Computerprogramm kann dabei eine Software mit einen Quellcode, der noch compiliert und gebunden oder der nur interpretiert werden muss, oder einen ausführbaren Softwarecode umfassen, der zur Ausführung nur noch in eine entsprechende Recheneinheit zu laden ist.

Des Weiteren geht die Erfindung aus von einem computerlesbaren Datenträger, welcher ein Programm umfasst, das zu einer Ausführung eines Verfahrens zu einer Unterstützung einer Atmung eines Patienten während einer medizinischen Bildgebungsuntersuchung vorgesehen ist.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine erfindungsgemäße medizinische Bildgebungsvorrichtung in einer schematischen Darstellung,
- Fig. 2: ein erfindungsgemäßes Verfahren zu einer Unterstützung einer Atmung eines Patienten während einer medizinischen Bildgebungsuntersuchung und
- Fig. 3.: eine Patientenschnittstelle der medizinischen Bildgebungsvorrichtung mit einem bildlichen Objekt.

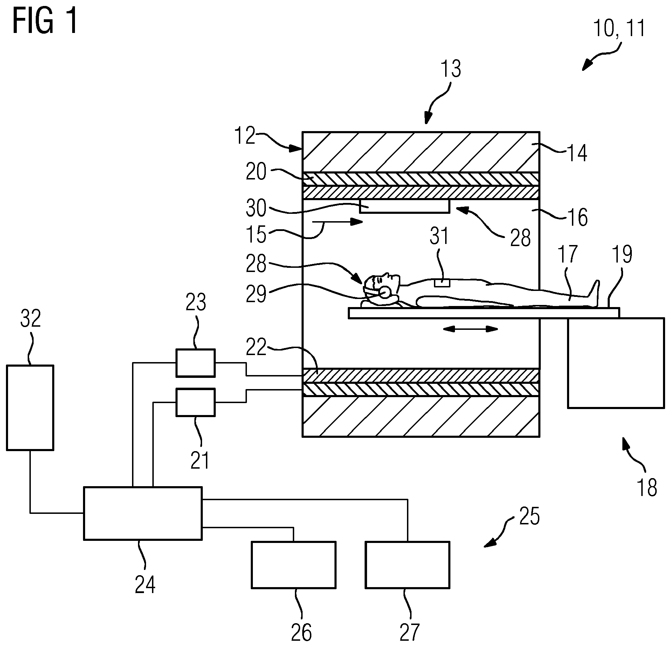

In der Fig. 1 ist eine medizinische Bildgebungsvorrichtung 10 schematisch dargestellt. Die medizinische Bildgebungsvorrichtung 10 ist im vorliegenden Ausführungsbeispiel von einer Magnetresonanzvorrichtung 11 gebildet, wobei beispielshaft die vorliegende Erfindung anhand der Magnetresonanzvorrichtung 11 erläutert wird. Die vorliegende Erfindung ist jedoch nicht auf Ausgestaltung der medizinischen Bildgebungsvorrichtung 10 auf eine Magnetresonanzvorrichtung 11 beschränkt und weitere Ausgestaltungen der medizinischen Bildgebungsvorrichtung 10 sind jederzeit denkbar.

Die Magnetresonanzvorrichtung 11 umfasst eine von einer Magneteinheit 12 gebildeten Scannereinheit 13. Die Magneteinheit 12 umfasst einen supraleitenden Hauptmagneten 14 zu einem Erzeugen eines starken und insbesondere konstanten Hauptmagnetfelds 15. Zudem weist die Magnetresonanzvorrichtung 11 einen Patientenaufnahmebereich 16 auf zu einer Aufnahme eines Patienten 17. Der Patientenaufnahmebereich 16 im vorliegenden Ausführungsbeispiel ist zylinderförmig ausgebildet und in einer Umfangsrichtung von der Magneteinheit 12 zylinderförmig umgeben. Grundsätzlich ist jedoch eine davon abweichende Ausbildung des Patientenaufnahmebereichs 16 jederzeit denkbar. Der Patient 17 kann mittels einer Patientenlagerungsvorrichtung 18 der Magnetresonanzvorrichtung 11 in den Patientenaufnahmebereich 17 geschoben und/oder gefahren werden. Die Patientenlagerungsvorrichtung 18 weist hierzu einen innerhalb des Patientenaufnahmebereichs 16 bewegbar ausgestalteten Patiententisch 19 auf.

Die Magneteinheit 12 weist weiterhin eine Gradientenspuleneinheit 20 zu einer Erzeugung von Magnetfeldgradienten auf, die für eine Ortskodierung während einer Bildgebung verwendet werden. Die Gradientenspuleneinheit 20 wird mittels einer Gradientensteuereinheit 21 der Magnetresonanzvorrichtung 11 gesteuert. Die Magneteinheit 12 umfasst weiterhin eine Hochfrequenzantenneneinheit 22 zu einer Anregung einer Polarisation, die sich in dem von dem Hauptmagneten 14 erzeugten Hauptmagnetfeld 15 einstellt. Die Hochfrequenzantenneneinheit 22 wird von einer Hochfrequenzantennensteuereinheit 23 der Magnetresonanzvorrichtung 11 gesteuert und strahlt hochfrequente Magnetresonanzsequenzen in einen Untersuchungsraum ein, der im Wesentlichen von einem Patientenaufnahmebereich 16 der Magnetresonanzvorrichtung 11 gebildet ist.

Zu einer Steuerung des Hauptmagneten 14, der Gradientensteuereinheit 21 und zur Steuerung der Hochfrequenzantennensteuereinheit 23 weist die Magnetresonanzvorrichtung 11 eine Systemsteuereinheit 24 auf. Die Systemsteuereinheit 24 steuert zentral die Magnetresonanzvorrichtung 11, wie beispielsweise das Durchführen einer vorbestimmten bildgebenden Gradientenechosequenz. Zudem umfasst die Systemsteuereinheit 24 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 11 eine Benutzerschnittstelle 25, die mit der Systemsteuereinheit 24 verbunden ist. Steuerinformationen wie beispielsweise Bildgebungsparameter, sowie rekonstruierte Magnetresonanzbilder können auf einer Anzeigeeinheit 26, beispielsweise auf zumindest einem Monitor, der Benutzerschnittstelle für ein medizinisches Bedienpersonal angezeigt werden. Weiterhin weist die Benutzerschnittstelle 25 eine Eingabeeinheit 27 auf, mittels der Informationen und/oder Parameter während eines Messvorgangs von dem medizinischen Bedienpersonal eingegeben werden können.

Die Magnetresonanzvorrichtung 11 umfasst zudem eine Patientenschnittstelle 28 für eine Kommunikation mit dem Patienten 17 während einer medizinischen Bildgebungsuntersuchung, insbesondere während einer Magnetresonanzuntersuchung. Die Patientenschnittstelle 28 umfasst im vorliegenden Ausführungsbeispiel eine visuelle Patientenschnittstelle 28. Zudem umfasst die Patientenschnittstelle 28 im vorliegenden Ausführungsbeispiel auch eine auditive und/oder akustische Patientenschnittstelle 28. Die Patientenschnittstelle 28 kann in einer alternativen Ausgestaltung der Erfindung auch nur eine visuelle Patientenschnittstelle 28 oder auch nur eine auditive und/oder akustische Patientenschnittstelle 28 umfassen.

Die auditive und/oder akustische Patientenschnittstelle 28 umfasst einen Kopfhörer 29, den der Patient 17 während der medizinischen Bildgebungsuntersuchung, insbesondere während der Magnetresonanzuntersuchung, trägt. Zudem kann die auditive und/oder akustische Patientenschnittstelle 28 auch einen Lautsprecher umfassen. Die visuelle Patientenschnittstelle 28 umfasst ein Display und/oder einen Monitor, der innerhalb des Patientenaufnahmebereichs 16 angeordnet ist. Vorzugsweise ist das Display außerhalb des Patientenaufnahmebereichs 16 angeordnet und wird mittels eines Spiegels 30 in den Patientenaufnahmebereich 16 projiziert. Vorzugsweise ist der Spiegel 30 Monitor an einer dem Patienten 17 gegenüberliegenden Wand des Patientenaufnahmebereichs 16 angeordnet. Zudem kann es auch sein, dass auch das Display und/oder der Monitor an einer dem Patienten 17 gegenüberliegenden Wand des Patientenaufnahmebereichs 16 angeordnet ist. Die visuelle Patientenschnittstelle 28 kann in einer alternativen Ausgestaltung der Erfindung auch eine Videobrille und/oder eine Projektionsfläche einer Projektionseinheit und/oder weitere visuelle Ausgabeeinheiten umfassen.

Die medizinische Bildgebungsvorrichtung 10, insbesondere die Magnetresonanzvorrichtung 11, umfasst im vorliegenden Ausführungsbeispiel zudem einen Atemsensor 31, der zur Erfassung eines Atemsignals des Patienten 17 ausgelegt ist.

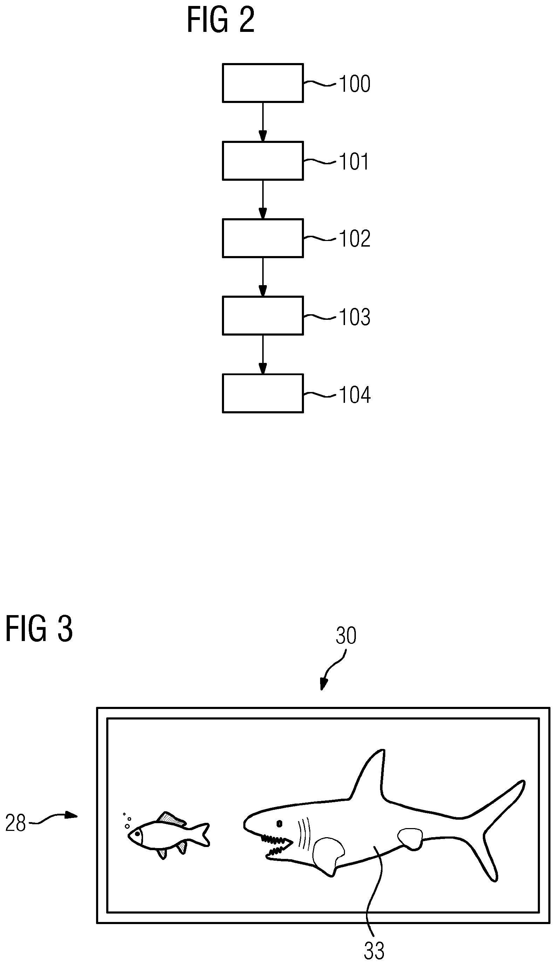

In Fig. 2 ist das erfindungsgemäße Verfahren zu einer Unterstützung einer Atmung des Patienten 17 während der medizinischen Bildgebungsuntersuchung, insbesondere der Magnetresonanzuntersuchung, dargestellt. Das Verfahren wird von einer Steuereinheit 32 zusammen mit der medizinischen Bildgebungsvorrichtung 10, insbesondere der Magnetresonanzvorrichtung 11, ausgeführt. Hierzu weist die Steuereinheit 32 eine Prozessoreinheit und eine Software und/oder Computerprogramme auf, die bei einer Ausführung durch die Prozessoreinheit das Verfahren zu einer Unterstützung einer Atmung eines Patienten 17 während einer medizinischen Bildgebungsuntersuchung ausführen. Die Software und/oder Computerprogramme können in einer Speichereinheit der Steuereinheit 32 gespeichert sein. Zudem können die Software und/oder die Computerprogramme auch in einer nicht näher dargestellten externen Speichereinheit gespeichert sein, wobei die Steuereinheit 32 mittels eines Datennetzes auf die Software und/oder Computerprogramme zugreifen kann.

Die Steuereinheit 32 kann zudem von der Magnetresonanzvorrichtung 11 umfasst sein. Zudem ist es auch denkbar, dass die Steuereinheit 32 separat zur Magnetresonanzvorrichtung 11 ausgebildet ist und mit der Magnetresonanzvorrichtung 11 mittels eines Datennetzes kommuniziert. Beispielsweise kann die Steuereinheit in einer Cloud integriert sein.

In einem ersten Verfahrensschritt 100 des erfindungsgemäßen Verfahrens wird zumindest ein Atemsignal des Patienten 17 während der medizinischen Bildgebungsuntersuchung, insbesondere der Magnetresonanzuntersuchung, erfasst. Hierzu befindet sich der Patient 17 auf dem Patiententisch 19 liegend innerhalb des Patientenaufnahmebereichs 16. Die Erfassung des zumindest einen Atemsignals des Patienten 17 erfolgt mittels des Atemsensors 31. Zudem kann das Atemsignal auch anhand von Magnetresonanzdaten, insbesondere Magnetresonanzrohdaten, einer Navigatormessung zur Bewegungserfassung des Patienten 17 erfasst werden. Das zumindest eine erfasste Atemsignal wird anschließend an die Steuereinheit 32 übertragen. Das zumindest eine erfasste Atemsignal des Patienten 17 kann beispielsweise ein Signal eines Einatmens des Patienten 17 und/oder ein Signal eines Ausatmens des Patienten 17 und/oder einen eigeatmeten Zustand des Patienten 17 und/oder einen ausgeatmeten Zustand des Patienten 17 und/oder eine aktuelle Atemfrequenz des Patienten 17 und/oder einen aktuellen Atemzyklus des Patienten 17 usw. umfassen.

Mittels der Steuereinheit 32 erfolgt anschließend in einem weiteren Verfahrensschritt 101 ein Ermitteln einer Abweichung des erfassten Atemsignals von einem idealen Atemzustand für die medizinische Bildgebungsuntersuchung, insbesondere für die Magnetresonanzuntersuchung. Der ideale Atemzustand ist bevorzugt an die Magnetresonanzuntersuchung, die gerade an dem Patienten 17 vorgenommen werden soll, angepasst, so dass, würde der Patienten 17 mit dem idealen Atemzustand atmen, optimale Bilddaten, insbesondere Magnetresonanzdaten, erfasst würden.

In einem weiteren Verfahrensschritt 102 wird anhand der ermittelten Abweichung eine Feedbackinformation für den Patienten 17 generiert. Die Feedbackinformation kann dabei eine visuelle und/oder bildliche Feedbackinformation umfassen. Zudem kann die Feedbackinformation auch eine auditive und/oder akustische Feedbackinformation umfassen.

In einem weiteren Verfahrensschritt 103 erfolge eine Ausgabe der Feedbackinformation an den Patienten 17 mittels der Patientenschnittstelle 28. Die Ausgabe erfolgt hierbei ebenfalls während der medizinischen Bildgebungsuntersuchung, insbesondere der Magnetresonanzuntersuchung. Sofern die generierte Feedbackinformation eine visuelle und/oder bildliche Feedbackinformation umfasst, wird diese mittels der visuellen Patientenschnittstelle 28 an den Patienten 17 ausgegeben. Sofern die generieret Feedbackinformation eine auditive und/oder akustische Feedbackinformation umfasst, wird diese mittels der auditiven und/oder akustischen Patientenschnittstelle 28 an den Patienten 17 ausgeben.

Das Verfahren mit den Verfahrensschritten 100 - 103 wiederholt sich kontinuierlich, bis die medizinische Bildgebungsuntersuchung, insbesondere die Magnetresonanzuntersuchung, an dem Patienten 17 beendet ist. Dies ermöglicht es, dass der Patient 17 durch Änderung seiner Atmung die Feedbackinformation beeinflussen kann. Insbesondere erhält derart der Patient 17 ständig eine aktualisierte Feedbackinformation, die ihm hilft, seine Atmung an den idealen Atemzustand anzupassen.

Die generierte Feedbackinformation umfasst hierbei ein Objekt, das mittels der ermittelten Abweichung des erfassten Atemsignals von dem idealen Atemzustand und damit auch mittels des Atemsignals durch den Patienten 17 steuerbar ist. Dabei kann das Objekt 33 der Feedbackinformation aufgrund einer Änderung einer Atemgeschwindigkeit und/oder aufgrund einer Änderung einer Atemfrequenz und/oder aufgrund einer Änderung eines Atemzustands durch den Patienten 17 gesteuert werden. Insbesondere kann hierbei eine Eigenschaft des Objekts 33, sie beispielsweise eine Form und/oder ein Aussehen und/oder eine Geschwindigkeit des Objekts 33, der Feedbackinformation aufgrund einer Änderung einer Atemgeschwindigkeit des Patienten 17 und/oder aufgrund einer Änderung einer Atemfrequenz des Patienten 17 und/oder aufgrund einer Änderung eines Atemzustands des Patienten 17 gesteuert werden.

Durch die Feedbackinformation, insbesondere durch das steuerbare Objekt 33 der Feedbackinformation, kann der Patient 17 dazu animiert werden, seinen Atemzustand zu ändern, bis dieser im Wesentlichen mit dem idealen Atemzustand für die medizinische Bildgebungsuntersuchung, insbesondere die Magnetresonanzuntersuchung, übereinstimmt.

Am Beispiel der visuellen und/oder bildlichen Feedbackinformation ist dies in Fig. 3 dargestellt. Die visuelle und/oder bildliche Feedbackinformation umfasst ein Objekt 33, das beispielhaft einen Haifisch, der versucht einen kleineren Fisch zu fangen, umfasst. Dabei kann eine Geschwindigkeit und/oder eine Position des Objekts 33 mittels einer Änderung der Abweichung des erfassten Atemsignals von dem idealen Atemzustand und damit auch mittels eines geänderten Atemsignals gesteuert werden. Im vorliegenden Ausführungsbeispiel kann eine Schwimmgeschwindigkeit und/oder eine Schwimmhöhe des Haifischs dabei mittels einer Änderung der Abweichung des erfassten Atemsignals von dem idealen Atemzustand und damit auch mittels eines geänderten Atemsignals gesteuert werden. Durch Änderung der Atemgeschwindigkeit des Patienten 17 und/oder der Atemfrequenz des Patienten 17 und/oder des Atemzustands des Patienten 17 kann somit ein Schwimmverhalten des Haifischs beeinflusst werden. Eine Übereinstimmung des Atemzustands des Patienten 17 mit dem idealen Atemzustand liegt vor, wenn beispielsweise der Haifisch den kleineren Fisch gefangen hat.

Grundsätzlich sind weitere Ausgestaltungen des bildlichen und/oder visuellen Objekts 33 der visuellen und/oder bildlichen Feedbackinformation möglich. Beispielsweise kann das bildliche und/oder visuelle Objekt 33 auch einen Ball umfassen, der bei einer Annäherung des Atemzustands und/oder des Atemsignals des Patienten an den idealen Atemzustand in einer Schwebeposition gehalten werden kann. Bei einem Abweichen des Atemsignals des Patienten von dem idealen Atemzustand weicht auch der Ball von seiner Schwebeposition ab. Ein Ändern der Atemfrequenz und/oder der Atemamplitude und/oder des Atemzustands durch den Patienten 17 kann dabei eine Geschwindigkeit und/oder eine Bewegungsrichtung des bildlichen und/oder visuellen Objekts 33, insbesondere des Balls, der Feedbackinformation beeinflussen.

Die Feedbackinformation umfasst zudem eine auditive und/oder akustische Feedbackinformation, die ein auditives und/oder akustisches Objekt umfasst. Das auditive und/oder akustische Objekt umfasst beispielsweise einen Ton und/oder eine Tonfolge, wobei eine Frequenz des Tons und/oder der Tonfolge und/oder eine Amplitude des Tons und/oder der Tonfolge aufgrund der Atemgeschwindigkeit des Patienten 17 und/oder der Atemfrequenz des Patienten 17 und/oder des Atemzustands des Patienten 17 steuerbar ist. Beispielsweise kann der ausgegebene Ton und/oder die ausgegebene Tonfolge einen hohen Ton und/oder eine hohe Tonfolge umfassen, wenn die Atmung, insbesondere der Atemzustand, des Patienten 17 zu langsam bezüglich des idealen Atemzustands ist. Auch kann beispielsweise der ausgegebene Ton und/oder die ausgegebene Tonfolge einen tiefen Ton und/oder eine tiefe Tonfolge umfassen, wenn die Atmung, insbesondere der Atemzustand, des Patienten 17 zu schnell bezüglich des idealen Atemzustands ist. Stimmt der aktuelle Atemzustand des Patienten 17 mit dem idealen Atemzustand für die medizinische Bildgebungsuntersuchung überein, kann beispielsweise kein Ton mehr zu hören sein. Umfasst das akustische Objekt eine Tonfolge und/oder eine Melodie, kann auch ein Takt mit dem aktuellen Atemzustand des Patienten 17 geändert werden und/oder die eine Geschwindigkeit der Tonfolge und/oder Melodie geändert werden. Zudem sind weitere Ausgestaltungen des auditiven und/oder akustischen Objekts der auditiven und/oder akustischen Feedbackinformation möglich.

In Abhängigkeit des erfassten Atemsignals können in einem weiteren Verfahrensschritt 104 medizinische Bilddaten, insbesondere Magnetresonanzdaten, wie beispielsweise Rohdaten, während der medizinischen Bildgebungsuntersuchung, insbesondere der Magnetresonanzuntersuchung, erfasst werden. Hierbei wird von der Steuereinheit 32 anhand des erfassten Atemsignals ein Triggersignal für die Magnetresonanzuntersuchung generiert. Zusätzlich kann die Generierung des Triggersignals auch abhängig sein von der ermittelten Abweichung des Atemsignals von dem idealen Atemzustand der medizinischen Bildgebungsuntersuchung, insbesondere der Magnetresonanzuntersuchung. Beispielsweise kann es vorgesehen sein, dass nur bis zu einer maximal zulässigen Abweichung des erfassten Atemsignals des Patienten 17 von dem idealen Atemzustand die Steuereinheit 32 in Triggersignal für die medizinische Bildgebungsuntersuchung, insbesondere die Magnetresonanzuntersuchung, generiert.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zu einer Unterstützung einer Atmung eines Patienten während einer medizinischen Bildgebungsuntersuchung mittels einer medizinischen Bildgebungsvorrichtung umfassend die folgenden Schritte:
- Erfassen eines Atemsignals des Patienten während der medizinischen Bildgebungsuntersuchung,
- Ermitteln einer Abweichung des erfassten Atemsignals von einem idealen Atemzustand für die medizinische Bildgebungsvorrichtung,
- Generieren einer Feedbackinformation für den Patienten anhand der Abweichung und
- Ausgabe der Feedbackinformation an den Patienten mittels einer Patientenschnittstelle während der medizinischen Bildgebungsuntersuchung.

2. Verfahren nach Anspruch 1,
**dadurch gekennzeichnet, dass** das Atemsignal mittels eines Atemsensors erfasst wird.

3. Verfahren nach einem der vorhergehenden Ansprüche,
**dadurch gekennzeichnet, dass** das Atemsignal anhand eines empfangenen Bilddatensignals einer Navigatormessung erfasst wird.

4. Verfahren nach einem der vorhergehenden Ansprüche,
**dadurch gekennzeichnet, dass** die Feedbackinformation visuell ausgegeben wird.

5. Verfahren nach einem der vorhergehenden Ansprüche,
**dadurch gekennzeichnet, dass** die Feedbackinformation auditiv ausgegeben wird.

6. Verfahren nach einem der vorhergehenden Ansprüche,
**dadurch gekennzeichnet, dass** die Feedbackinformation ein Objekt umfasst, das mittels des Atemsignals steuerbar ist.

7. Verfahren nach Anspruch 6,
**dadurch gekennzeichnet, dass** das Objekt der Feedbackinformation aufgrund einer Atemgeschwindigkeit des Patienten und/oder einer Atemfrequenz des Patienten und/oder eines Atemzustands des Patienten steuerbar ist.

8. Verfahren nach einem der Ansprüche 6 bis 7,
**dadurch gekennzeichnet, dass** eine Eigenschaft des Objekts direkt vom Patienten durch Änderung seiner Atemgeschwindigkeit und/oder seiner Atemfrequenz und/oder seines Atemzustands veränderbar und/oder steuerbar ist.

9. Verfahren nach einem der Ansprüche 6 bis 8,
**dadurch gekennzeichnet, dass** das Objekt ein bildliches und/oder visuelles Objekt umfasst, das mittels der Patientenschnittstelle darstellbar ist und dessen Aussehen und/oder dessen Geschwindigkeit und/oder dessen Größe aufgrund der Atemgeschwindigkeit des Patienten und/oder der Atemfrequenz des Patienten und/oder des Atemzustands des Patienten steuerbar ist.

10. Verfahren nach einem der Ansprüche 6 bis 9,
**dadurch gekennzeichnet, dass** das Objekt einen Ton und/oder eine Tonfolge umfasst, wobei eine Frequenz und/oder eine Amplitude des Tons und/oder der Tonfolge aufgrund einer Atemgeschwindigkeit des Patienten und/oder einer Atemfrequenz des Patienten und/oder eines Atemzustands des Patienten steuerbar ist.

11. Verfahren nach einem der vorhergehenden Ansprüche,
**dadurch gekennzeichnet, dass** in Abhängigkeit des erfassten Atemsignals medizinischen Bilddaten während der medizinischen Bildgebungsuntersuchung erfasst werden.

12. Steuereinheit mit einer Prozessoreinheit, die zu einer Steuerung des Verfahrens zu einer Unterstützung einer Atmung eines Patienten während einer medizinischen Bildgebungsuntersuchung ausgelegt ist.

13. Medizinische Bildgebungsvorrichtung mit einer Scannereinheit zur Erfassung von medizinischen Bildgebungsdaten während einer medizinischen Bildgebungsuntersuchung, einem Atemsensor und einer Patientenschnittstelle, wobei die medizinische Bildgebungsvorrichtung zusammen mit einer Steuereinheit nach Anspruch 12 zu einer Ausführung eines Verfahrens zu einer Unterstützung einer Atmung eines Patienten während einer medizinischen Bildgebungsuntersuchung nach einem der Ansprüche 1 bis 11 ausgelegt ist.

14. Medizinische Bildgebungsvorrichtung nach Anspruch 13,
**dadurch gekennzeichnet, dass** die Patientenschnittstelle eine akustische Patientenschnittstelle umfasst.

15. Medizinische Bildgebungsvorrichtung nach einem der Ansprüche 13 bis 14,
**dadurch gekennzeichnet, dass** die Patientenschnittstelle eine visuelle Patientenschnittstelle umfasst.

16. Computerprogrammprodukt, welches ein Programm umfasst und direkt in einen Speicher einer programmierbaren Steuereinheit ladbar ist, mit Programmmitteln, um ein Verfahren zu einer Unterstützung einer Atmung eines Patienten während einer medizinischen Bildgebungsuntersuchung nach einem der Ansprüche 1 bis 11 auszuführen, wenn das Programm in der Steuereinheit ausgeführt wird.

17. Elektronisch lesbarer Datenträger mit darauf gespeicherten elektronisch lesbaren Steuerinformationen, welche derart ausgestaltet sind, dass sie bei Verwendung des Datenträgers in einer Steuereinheit einer Magnetresonanzvorrichtung das Verfahren zu einer Unterstützung einer Atmung eines Patienten während einer medizinischen Bildgebungsuntersuchung nach einem der Ansprüche 1 bis 11 durchführen.
